# EUROPEAN PATENT APPLICATION

(11) **EP 3 995 478 A1**
(43) Date of publication of application: **11.05.2022**
(21) Application number: 20834182.6
(22) Date of filing: 30.06.2020
(51) Int. Cl.: C07C 17/04, C07B 61/00, C07C 19/10, C09K 5/04

(54) **METHOD FOR PRODUCING ALKANE**

(30) Priority: 01.07.2019 JP 2019122744
(71) Applicant: Daikin Industries, Ltd., Osaka-shi, Osaka 530-8323 (JP)
(72) Inventor: ETOU, Yuusuke, Osaka-shi, Osaka 530-8323 (JP); NAKAMURA, Shingo, Osaka-shi, Osaka 530-8323 (JP)
(74) Representative: Hoffmann Eitle
(86) International application number: PCT/JP2020/025617
(87) International publication number: WO 2021/002344

(57) **Abstract**

An object of the present disclosure is to provide a chlorinated alkane at a high conversion rate (yield) and with high selectivity. Provided is a method for producing an alkane, the method including the step of subjecting an alkene to a chlorination reaction, the chlorination reaction step being performed using zeolite as a catalyst.

## Description

### Technical Field

The present disclosure relates to a method for producing an alkane.

### Background Art

Patent Literature (PTL) 1 discloses a method for producing an alkane (2,3-dichloro-1,1,1,4,4,4-hexafluorobutane, HCFC-336mdd) by subjecting an alkene ((E)-1,1,1,4,4,4-hexafluoro-2-butene, E-1336mzz) to a chlorination reaction using activated carbon as a catalyst.

### Citation List

### Patent Literature

PTL 1: WO2015/142981A1

### Summary of Invention

### Technical Problem

An object of the present disclosure is to produce a chlorinated alkane at a high conversion rate (yield) and a high selectivity.

### Solution to Problem

The present disclosure includes the following subject matter.

### Item 1.

A method for producing an alkane represented by formula (1): 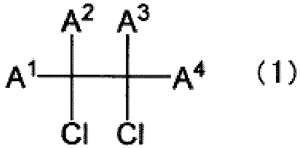
(wherein A¹, A², A³, and A⁴ are the same or different and represent a hydrogen atom, a halogen atom, a hydrocarbon group, or a perfluoroalkyl group),
the method comprising the step of subjecting an alkene represented by formula (2): 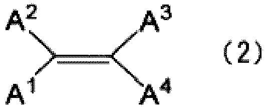
(wherein A¹, A², A³, and A⁴ are as defined above) to a chlorination reaction, the chlorination reaction step being performed using a zeolite as a catalyst.

### Item 2.

The production method according to Item 1, wherein the chlorination reaction is performed in a gas phase.

### Item 3.

A composition comprising
an alkane represented by formula (1): 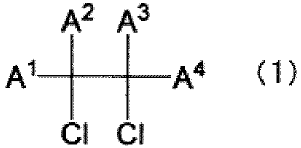
(wherein A¹, A², A³, and A⁴ are the same or different and represent a hydrogen atom, a halogen atom, a hydrocarbon group, or a perfluoroalkyl group); and
at least one additional compound comprising (selected from the group consisting of) a hydrochlorofluorocarbon (hydrochlorofluorocarbons) (HCFC) compound containing at least one chlorine (excluding the alkane represented by formula (1)).

### Item 4.

The composition according to Item 3, wherein the alkane represented by formula (1) is present in an amount of 90 mol% or more and the additional compound is present in an amount of 10 mol% or less, based on the total amount of the composition taken as 100 mol%.

### Item 5.

The composition according to Item 3 or 4, wherein the additional compound is at least one member selected from the group consisting of monochlorohexafluorobutane, trichlorohexafluorobutane, and tetrachlorohexafluorobutane.

### Item 6.

The composition according to any one of Items 3 to 5, wherein the composition is for use as an etching gas, a refrigerant, or a heat transfer medium.

### Advantageous Effects of Invention

The present disclosure enables the production of a chlorinated alkane at a high conversion rate (yield) and with a high selectivity.

### Description of Embodiments

The present inventors conducted extensive research and found that an alkane represented by formula (1) can be produced at a high conversion rate (yield) and with a high selectivity by subjecting an alkene as a starting material compound to a chlorination reaction using zeolite as a catalyst.

The present inventors conducted further research on the basis of this finding and completed the present disclosure.

The present disclosure includes the following embodiments.

The method for producing an alkane represented by formula (1): 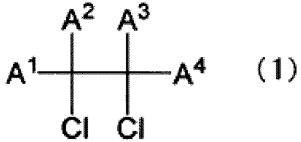
(wherein A¹, A², A³, and A⁴ are the same or different and represent a hydrogen atom, a halogen atom, a hydrocarbon group, or a perfluoroalkyl group) according to the present disclosure comprises the step of subjecting an alkene represented by formula (2) : 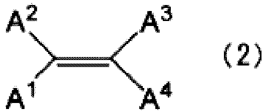
(wherein A¹, A², A³, and A⁴ are as defined above) to a chlorination reaction.

In the present disclosure, the step of the chlorination reaction is performed using zeolite as a catalyst.

According to a preferred embodiment of the present disclosure, the chlorination reaction is performed in a gas phase.

In the present disclosure, a chlorinated alkane can be produced at a high conversion rate (yield) and with a high selectivity by satisfying the requirements described above.

In the present disclosure, the "conversion rate" refers to the percentage (mol%) of the total molar amount of the compounds (e.g., a chlorinated alkane etc.) other than the starting material compound (an alkene) that are present in a gas flowing out from a reactor outlet relative to the molar amount of the starting material compound supplied to a reactor.

In the present disclosure, "selectivity" refers to the percentage (mol%) of the total molar amount of the target compound (a chlorinated alkane) present in a gas flowing out from a reactor outlet relative to the total molar amount of the compounds other than the starting material compound (e.g., a chlorinated alkane etc.) in the gas flowing out from the reactor outlet.

The method for producing an alkane according to the present disclosure can increase the yield of the target compound by performing a chlorination reaction using zeolite as a catalyst, for example, in a gas phase, unlike conventional methods in which the reaction is performed using activated carbon as a catalyst.

### (1) Starting Material Compound

In the present disclosure, the starting material compound is an alkene represented by formula (2): 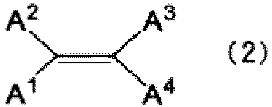 (wherein A¹, A², A³, and A⁴ are the same or different and represent a hydrogen atom, a halogen atom, a hydrocarbon group, or a perfluoroalkyl group).

In formula (2), A¹, A², A³, and A⁴ are the same or different and represent a hydrogen atom, a halogen atom, a hydrocarbon group, or a perfluoroalkyl group.

Examples of halogen atoms represented by A¹, A², A³, and A⁴ include a fluorine atom, a chlorine atom, a bromine atom, and an iodine atom. The halogen atom represented by A¹, A², A³, and A⁴ is preferably a fluorine atom.

The perfluoroalkyl group represented by A¹, A², A³, and A⁴ is an alkyl group in which all of the hydrogen atoms are replaced with a fluorine atom. The perfluoroalkyl group has, for example, 1 to 20 carbon atoms, preferably 1 to 12 carbon atoms, more preferably 1 to 6 carbon atoms, still more preferably 1 to 4 carbon atoms, and particularly preferably 1 to 3 carbon atoms. The perfluoroalkyl group is preferably a linear or branched perfluoroalkyl group. The perfluoroalkyl group is preferably a trifluoromethyl group (CF₃-) or a pentafluoroethyl group (C₂F₅-).

The hydrocarbon group represented by A¹, A², A³, and A⁴ has, for example, 1 to 20 carbon atoms, preferably 1 to 12 carbon atoms, more preferably 1 to 6 carbon atoms, still more preferably 1 to 4 carbon atoms, and particularly preferably 1 to 3 carbon atoms. The hydrocarbon group is preferably a linear or branched hydrocarbon group. The hydrocarbon group is preferably a trifluoromethyl group (CF₃-) or a pentafluoroethyl group (C₂F₅-) .

A¹, A², A³, and A⁴ are the same or different and are preferably a hydrogen atom, a fluorine atom, or a trifluoromethyl group (CF₃-) .

In the present disclosure, the starting material compound is a compound represented by formula (2) wherein cyclic structures such as a benzene ring are excluded from the hydrocarbon group represented by A¹, A², A³, and A⁴.

The alkene represented by formula (2), which is a starting material compound, is preferably a compound having 2 carbon atoms (a C₂ compound) to a compound having 8 carbon atoms (a C₈ compound), more preferably a C₂ compound to a C₄ compound, and particularly preferably a C₄ compound, from the standpoint of being able to produce a chlorinated alkane with a higher conversion rate (yield) and with a higher selectivity using zeolite.

The starting material compound alkene represented by formula (2) is preferably an alkene wherein A¹, A², A³, and A⁴ are the same or different and represent a hydrogen atom or a trifluoromethyl group (CF₃-) from the standpoint of being able to produce a chlorinated alkane at a high conversion rate (yield) and with a high selectivity.

Examples of the alkene represented by formula (2), which is a starting material compound, include compounds represented by 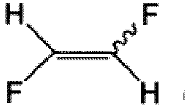 (Cis or trans isomer, (Z) or (E)-HFO-1132) 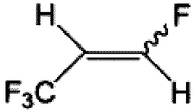 (Cis or trans isomer, (Z) or (E)-1234ze) 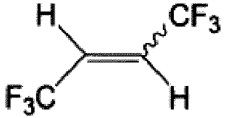 (Cis or trans isomer, (Z) or (E)-1336mzz) 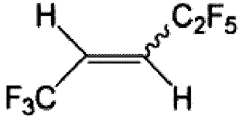 (Cis or trans isomer, (Z) or (E)-1538mzz) 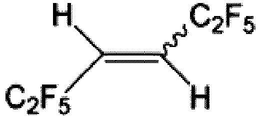 (Cis or trans isomer)

and the like.

These alkenes represented by formula (2) can be used alone or in a combination of two or more. Commercially available products can also be used as such alkenes.

### (2) Chlorination reaction

The chlorination reaction step in the present disclosure is performed using zeolite as a catalyst.

In the step of the chlorination reaction in the present disclosure, for example, the starting material compound alkene represented by formula (2) is preferably a compound wherein A¹, A², A³, and A⁴ are the same or different and represent a hydrogen atom, a fluorine atom, or a trifluoromethyl group (CF₃-) from the standpoint of being able to produce a chlorinated alkane at a high conversion rate (yield) and with a high selectivity.

The chlorination reaction step in the present disclosure is a chlorination reaction according to the following reaction scheme.

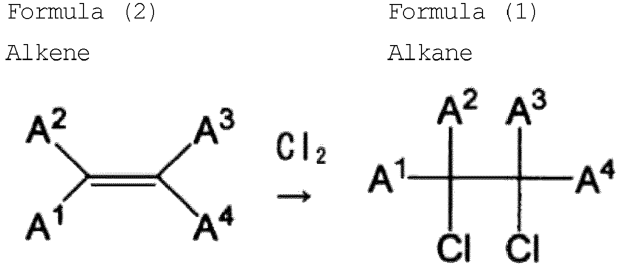

In formulas (1) and (2), A¹, A², A³, and A⁴ are the same or different and represent a hydrogen atom, a halogen atom, a hydrocarbon group, or a perfluoroalkyl group.

The chlorination reaction step in the present disclosure is preferably a chlorination reaction according to the following reaction scheme.

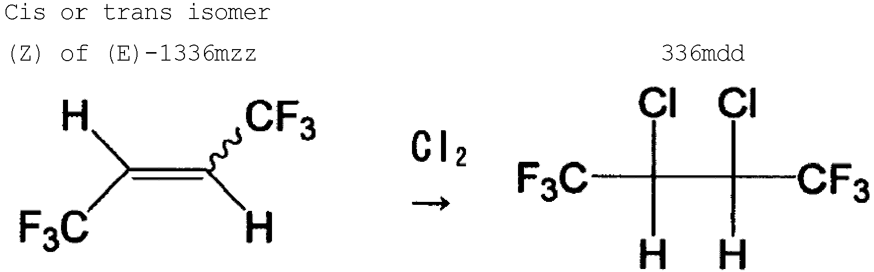

### Zeolite catalyst

In the chlorination reaction step in the present disclosure, the chlorination reaction is performed in the presence of a zeolite catalyst. Zeolites are one of the clay minerals. Zeolites are hydrous aluminosilicates that contain alkali or alkaline earth metals and that are composed of a rigid anionic framework with regular channels (tubular pores) and cavities (cavities).

Zeolites are typically represented by the composition of (M^{I},M^{II}_{1/2})ₘ(AlₘSiₙO₂₍ₘ₊ₙ₎)·ₓH₂O (n≥m) (M^{I}: Li⁺, Na⁺, K⁺, etc., M^{II}: Ca²⁺, Mg²⁺, Ba²⁺, etc.) . The cations compensate for the negative charge of the aluminosilicate framework.

There is no limitation on the cation in the zeolite; H⁺, Li⁺, Na⁺, K, Ca²⁺, Mg²⁺, Ba²⁺, and the like are usually used.

The basic unit of the structure is a tetrahedral structure of SiO₄ or AlO₄ (TO₄ tetrahedron in combination). These structures are infinitely connected in three-dimensional directions to form crystals. The crystals of zeolites are porous, and typically have a pore diameter of about 0.2 to 1.0 nm (2Å to 10Å). Zeolites have a molecular sieving effect (molecular sieve), which means that a molecule having a size larger than the pore size of zeolites cannot enter. Zeolites have properties such as solid acidity, ion exchange capacity, catalytic capacity, and adsorption capacity, in addition to the molecular sieving effect due to the pore derived from their framework structure.

In the chlorination reaction step in the present disclosure, an alkene is chlorinated in the presence of a zeolite catalyst to produce a chlorine-containing alkane. The chlorination reaction step in the present disclosure comprises a step of bringing an alkene into contact with zeolite. In the present disclosure, bringing the alkene in contact with zeolite means allowing the alkene to pass through a column or the like filled with zeolite, or introducing an alkene into a container filled with zeolite in the presence of chlorine (Cl₂).

In the present disclosure, zeolite may be treated for activation before its use. The activation treatment conditions may be, for example, a drying treatment by heating in a vacuum (10⁻¹ mmHg to 10⁻³ mmHg) at a temperature in the range of 300°C to 600°C overnight. In the present disclosure, zeolites that do not undergo the activation treatment can also be suitably used.

The zeolite used in this disclosure is preferably porous.

The zeolite used in this disclosure is commercially available.

### Properties of Zeolite

In the present disclosure, there is no limitation on the form of the zeolite to be used. The alkene may pass through a device filled with the zeolite; or the alkene may be introduced into a device filled with the zeolite, and the chlorinated alkane may be removed after a predetermined amount of time has passed.

In the present disclosure, the zeolite to be used may be in the form of powders, granules, or pellets; or may be used as a molded body. From an industrial perspective, the zeolite to be used is preferably a molded body. Although there is no limitation on the shape of the molded body, a cylindrical shape having a diameter of about 0.5 to 5 mm and a length of about 1 to 15 mm, or a spherical shape having a diameter of about 0.5 to 10 mm is preferably used.

In the present disclosure, there is no limitation on the method for producing the molded body of the zeolite. For example, a conventional known method using kaolin clay as a binder can be used.

### Silica/alumina Ratio (SiO₂/Al₂O₃ ratio) of Zeolite

In the present disclosure, a zeolite with a SiO₂/Al₂O₃ ratio (molar ratio) of 1 to 10 is preferably used from the standpoint of being able to efficiently produce a chlorinated alkane at a high conversion rate (yield) and with a high selectivity. In the present disclosure, the SiO₂/Al₂O₃ratio (molar ratio) of the zeolite used is more preferably 1.5 to 9, and still more preferably 2 to 7.

In the present disclosure, a zeolite with a SiO₂/Al₂O₃ ratio (molar ratio) of 1 to 10 is preferably used because a SiO₂/Al₂O₃ratio (molar ratio) smaller than 1 increases the polarity of the zeolite, whereas a SiO₂/Al₂O₃ratio larger than 10 decreases the polarity of the zeolite.

### Pore Size of Zeolite

In the present disclosure, a zeolite having an average pore size of 6Å to 15Å is preferably used from the standpoint of being able to efficiently chlorinate an alkene and produce a chlorinated alkane at a high conversion rate (yield) and with a high selectivity. In the present disclosure, the average pore size of the zeolite used is more preferably 7Å to 12Å, and particularly preferably the zeolite has an average pore size of 8Å to 10Å.

In the present disclosure, based on using zeolite having an average pore size of 6Å to 15Å, when the starting material alkene is a compound preferably having 2 carbon atoms (a C₂ compound) to 8 carbon atoms (a C₈ compound) , and more preferably a compound having a C₂ to C₄ compound, an alkene can be efficiently chlorinated, thus producing a chlorinated alkane at a high conversion rate (yield) and with a high selectivity.

### Cationic Species of Zeolite

In the present disclosure, from the standpoint of being able to efficiently chlorinate an alkene and produce a chlorinated alkane at a high conversion rate (yield) and with a high selectivity, cation species (cationic species) of the zeolite are usually preferably H⁺, Li⁺, Na⁺, K⁺, Ca²⁺, Mg²⁺, Ba²⁺, and the like. Zeolites whose cation species are H⁺, Na⁺, or the like are particularly preferably used.

In the present disclosure, cations in the zeolite compensate for the negative charge of the aluminosilicate framework, and an alkene can be efficiently chlorinated, and a chlorinated alkane can be produced at a high conversion rate (yield) and with a high selectivity.

### Specific Surface Area of Zeolite

The specific surface area of zeolite as measured by the BET method (hereinafter also referred to as BET specific surface area) is preferably 50 m²/g to 3,000 m²/g, and more preferably 100 m²/g to 1,000 m²/g, still more preferably 200 m²/g to 800 m²/g, and particularly preferably 250 m²/g to 700 m²/g, from the standpoint of being able to efficiently subject an alkene to a chlorination reaction and produce a chlorinated alkane at a high conversion rate (yield) and with a high selectivity.

In the present disclosure, when the BET specific surface area of the zeolite catalyst is in the range described above, the density of the zeolite particles is not overly small and the target compound can thus be obtained with a high selectivity. It is also possible to increase the conversion rate of the starting material compound.

### Amount of Chlorine Used (Cl₂/Alkene Molar Ratio)

In the present disclosure, the amount of chlorine used is not particularly limited. In the present disclosure, from the standpoint of being able to efficiently subject an alkene to a chlorination reaction and produce a chlorinated alkane at a high conversion rate (yield) and with a high selectivity, the chlorine is preferably used in an amount of 0.1 to 10 moles (Cl₂/alkene molar ratio is 0.1 to 10), more preferably 1 to 5 moles (Cl₂/alkene molar ratio is 1 to 5), more preferably 1.5 to 3 moles (Cl₂/alkene molar ratio is 1.5 to 3), and particularly preferably 2 moles (Cl₂/alkene molar ratio is 2), per mole of the alkene.

### Gas-phase Reaction

According to a preferred embodiment of the present disclosure, the chlorination reaction is performed in a gas phase. According to a particularly preferred embodiment of the present disclosure, the chlorination reaction is performed in a gas phase, and particularly preferably by a gas-phase continuous flow process using a fixed-bed reactor. Performing the chlorination reaction by a gas-phase continuous flow process can simplify equipment, operations, etc., and is also economically advantageous.

### Chlorination Reaction Temperature

In the chlorination reaction of an alkene in the present disclosure, there is no limitation on the temperature at which chlorine is added and an alkene is brought into contact with zeolite.

In the chlorination reaction step in the present disclosure, the lower limit of the reaction temperature is usually 50°C, and preferably 100°C, from the standpoint of allowing a chlorination reaction to more efficiently proceed to obtain the target compound with a higher selectivity and from the standpoint of suppressing a decrease in conversion rate.

In the chlorination reaction step in the present disclosure, the upper limit of the reaction temperature is usually 600°C, preferably 500°C, more preferably 300°C, and particularly preferably 200°C, from the standpoint of allowing a chlorination reaction to more efficiently proceed to obtain the target compound with a higher selectivity and from the standpoint of suppressing a decrease in selectivity due to decomposition or polymerization of the reaction product.

### Chlorination Reaction Time

In the chlorination reaction of the alkene of the present disclosure, there is no limitation on the time period during which chlorine is added and the alkene is in contact with zeolite.

Regarding the chlorination reaction time, as the contact time (W/F₀) [W: weight of metal catalyst (g), F₀: flow rate of starting material compound (cc/sec)] between the starting material compound (alkene) and the zeolite catalyst increases, the conversion rate of the starting material compound increases. However, the amount of the zeolite catalyst used increases, which requires larger equipment and is thus inefficient.

Therefore, the chlorination reaction time in the present disclosure is preferably such that the contact time of the starting material compound with the zeolite catalyst (W/F₀) is 1 g·sec/cc to 200 g·sec/cc, more preferably 5 g·sec/cc to 100 g·sec/cc, even more preferably 8 g·sec/cc to 50 g·sec/cc, and particularly preferably 10 g·sec/cc to 30 g·sec/cc, from the viewpoint of increasing the conversion rate of the starting material compound (alkene) and suppressing the equipment cost.

The contact time between the starting material compound and the catalyst refers to the time period during which the starting material compound (alkene) and the zeolite catalyst are in contact with each other.

When the chlorination reaction in the present disclosure is performed in the presence of the catalyst in a gas phase, the target compound can be obtained with a higher selectivity by adjusting the reaction temperature and the reaction time (contact time), particularly so as to be suitable for the catalyst used.

### Pressure in Chlorination Reaction

In the chlorination reaction of an alkene in the present disclosure, there is no limitation on the pressure at which chlorine is added and the alkene is in contact with zeolite.

In the present disclosure, the reaction pressure for the chlorination reaction is, for example, preferably -0.05 MPa to 2 MPa, more preferably -0.01 MPa to 1 MPa, and still more preferably ambient pressure to 0.5 MPa, from the standpoint of allowing a chlorination reaction to proceed more efficiently. In the present disclosure, when the pressure is not indicated, gauge pressure is used.

In the chlorination reaction in the present disclosure, the reactor in which chlorine (Cl₂) is added to the starting material compound and the alkene is brought into contact with a zeolite catalyst and allowed to react is not limited in terms of shape and structure as long as the reactor is resistant to temperature and pressure. Examples of reactors include vertical reactors, horizontal reactors, multi-tube reactors, and the like. Examples of reactor materials include glass, stainless steel, iron, nickel, iron nickel alloy, and the like.

### Examples of Chlorination Reaction

The chlorination reaction in the present disclosure can be performed in flow mode in which a starting material compound (alkene) is continuously fed to a reactor, chlorine (Cl₂) is added, and the target compound (chlorinated alkane) is continuously taken out from the reactor, or can be performed in batch mode. Since the target compound remaining in the reactor allows the chlorination reaction to further proceed, the chlorination reaction is preferably performed in flow mode.

In the present disclosure, the chlorination reaction step is preferably performed in a gas phase, and particularly preferably by a gas-phase continuous distribution process using a fixed-bed reactor. Performing the chlorination reaction by a gas-phase continuous flow process can simplify equipment, operations, etc., and is also economically advantageous.

In the present disclosure, from the standpoint of suppressing the degradation of the zeolite catalyst, the atmosphere in which the chlorination reaction is performed is preferably an atmosphere in which inert gas and/or hydrogen fluoride is present. The inert gas is preferably at least one member selected from the group consisting of nitrogen, helium, argon, and carbon dioxide. Of these inert gasses, nitrogen is more preferable from the standpoint of reducing costs. The concentration of the inert gas is preferably 0 to 50 mol% of the gas component introduced into the reactor.

In the present disclosure, after completion of the chlorination reaction, a purification treatment can be optionally performed in accordance with an ordinary method to obtain the chlorinated alkane represented by formula (1).

### (3) Target Compound

The target compound in the present disclosure is an alkane (chlorinated alkane) represented by formula (1): 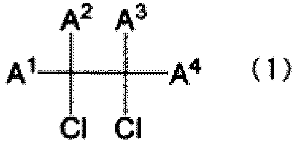 (wherein A¹, A², A³, and A⁴ are the same or different and represent a hydrogen atom, a halogen atom, a hydrocarbon group, or a perfluoroalkyl group).

In formula (1), A¹, A², A3 and A⁴ are the same or different and represent a hydrogen atom, a halogen atom, a hydrocarbon group, or a perfluoroalkyl group.

The target compound alkane represented by formula (1) is preferably a compound wherein A¹, A², A³, and A⁴ are the same or different and represent a hydrogen atom, a fluorine atom, or a trifluoromethyl group (CF₃-) .

Examples of the alkane represented by formula (1) to be produced include the compounds represented by the following: 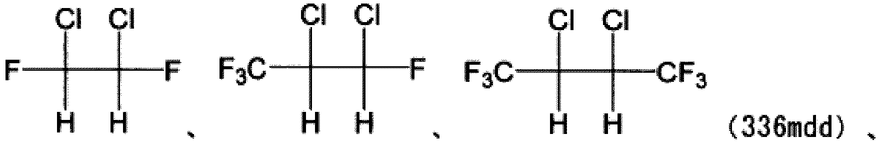 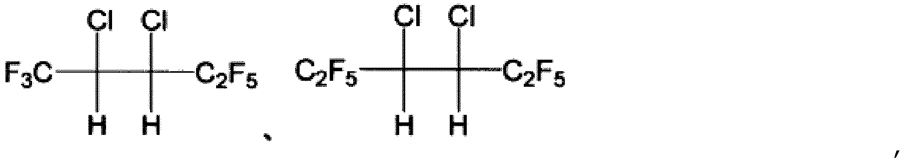 and the like.

In the method for producing an alkane according to the present disclosure, an alkene represented by formula (2) is used as a starting material compound; chlorine is added; and a chlorination reaction is allowed to proceed in the presence of a zeolite catalyst to produce an alkane represented by formula (1).

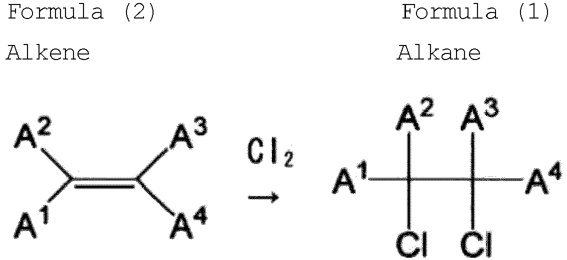

In formulae (1) and (2), A¹, A², A³, and A⁴ are the same or different and represent a hydrogen atom, a halogen atom, a hydrocarbon group, or a perfluoroalkyl group.

In the chlorination reaction step in the present disclosure, the starting material compound alkene represented by formula (2) is preferably a compound wherein A¹, A², A³ and A⁴ are the same or different and represent a hydrogen atom or a trifluoromethyl group (CF₃-). The chlorination reaction is preferably performed according to the following reaction scheme:

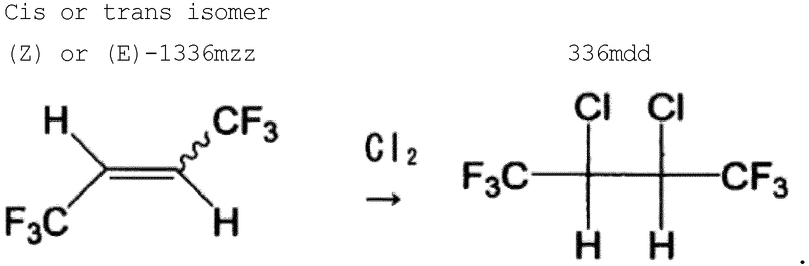

### (4) Composition Containing Alkane

In the manner described above, an alkane represented by formula (1) (chlorinated alkane) can be obtained. However, as described above, an alkane represented by formula (1) may also be obtained in the form of a composition containing an alkane represented by formula (1) and an alkene represented by formula (2) .

The alkane represented by formula (1) contained in the composition is preferably an alkane wherein A¹, A², A³ and A⁴ are the same or different and represent a hydrogen atom, a fluorine atom, or a trifluoromethyl group (CF₃-).

In the composition containing the alkane represented by formula (1) of the present disclosure, the alkane represented by formula (1) is preferably present in an amount of 95 mol% or more, and more preferably 99 mol% or more, based on the total amount of the composition taken as 100 mol%. In the composition containing the alkane represented by formula (1) of the present disclosure, the alkane represented by formula (1) is preferably present in an amount of 80 mol% to 99.9 mol%, more preferably 90 mol% to 99.9 mol%, and still more preferably 95 mol% to 99.9 mol%, based on the total amount of the composition taken as 100 mol%.

The alkane composition represented by formula (1) of the present disclosure can contain an alkane represented by formula (1) and at least one additional compound comprising (selected from the group consisting of) a hydrochlorofluorocarbon (hydrochlorofluorocarbons) (HCFC) compound containing at least one chlorine (excluding the alkane represented by formula (1)).

The alkane represented by formula (1) is preferably present in an amount of 90 mol% or more and the additional compound is preferably present in an amount of 10 mol% or less, based on the total amount of the composition taken as 100 mol%.

The additional compound is preferably at least one member selected from the group consisting of monochlorohexafluorobutane, trichlorohexafluorobutane, and tetrachlorohexafluorobutane.

In the method for producing an alkane according to the present disclosure, 2,2,3,3-tetrachloro-1,1,1,4,4,4-hexafluorobutane (HCFC-316maa, CF₃CCl₂CCl₂CF₃) can be produced in the chlorination reaction.

In the composition containing an alkane represented by formula (1) according to the present disclosure, for example, CF₃CHClCHClCF₃ (336mdd) is present in an amount of 99 mol% or more, and CF₃CCl₂CCl₂CF₃ (316maa) is present in an amount of 1 mol% or less, based on the total amount of the composition taken as 100 mol%.

The production method according to the present disclosure can produce an alkane represented by formula (1) (chlorinated alkane) with a particularly high selectivity, even when the alkane represented by formula (1) is obtained in the form of a composition containing the alkane represented by formula (1). Thus, it is possible to reduce the amount of components other than the alkane represented by formula (1) in the composition. The production method according to the present disclosure can reduce the labor for purification to obtain the alkane represented by formula (1).

The composition containing an alkane represented by formula (1) of the present disclosure is preferably used as an etching gas for forming state-of-the-art microstructures, such as semiconductors and liquid crystals, as well as refrigerants or heat transfer media, as in the case of the alkane represented by formula (1) alone. The composition containing an alkane represented by formula (1) of the present disclosure can also be effectively used for various applications, such as deposit gases, building blocks for organic synthesis, cleaning gases, etc. as well as etching gases.

The deposit gases refer to gases for depositing an etching-resistant polymer layer.

The building blocks for organic synthesis refer to a substance that can serve as a precursor of a compound that has a highly reactive skeleton. For example, when the alkane represented by formula (1) according to the present disclosure or the composition containing the alkane represented by formula (1) is reacted with a fluorine-containing organic silicon compound such as CF₃Si(CH₃)₃, a fluoroalkyl group, such as CF₃ group, is introduced to convert the alkane into a substance that can be used as a cleaner or a fluorine-containing pharmaceutical intermediate.

The above describes embodiments of the present disclosure. However, various modifications of the embodiments and details can be made without departing from the spirit and scope of the claims.

### Examples

The following describes the present disclosure in more detail with reference to Examples. However, the present disclosure is not limited to these Examples.

### Examples: Zeolite Catalyst

In the alkane production methods in the Examples, the starting material compound used was an alkene represented by formula (2) wherein A¹, A², A³ and A⁴ are the same or different and represent a hydrogen atom or a trifluoromethyl group (CF₃-), and the alkene was subjected to a chlorination reaction in the presence of a zeolite catalyst by adding chlorine according to the following reaction scheme:

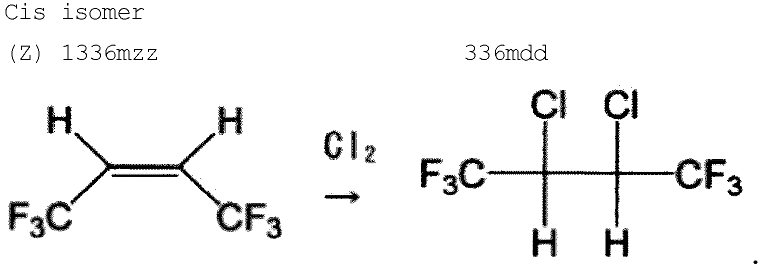

Starting material compound: (Z)1336mzz: (Z)-1,1,1,4,4,4-hexafluoro-2-butene

Target compound: HCFC-336mdd: 2,3-dichloro-1,1,1,4,4,4-hexafluorobutane

The above chlorination reaction can also produce 2,2,3,3-tetrachloro-1,1,1,4,4,4-hexafluorobutane (HCFC-316maa, CF₃CCl₂CCl₂CF₃) .

A zeolite catalyst was placed in a SUS pipe (outer diameter: 1/2 inch) used as a reaction tube. After the catalyst was dried at 500°C for 3 hours in a nitrogen atmosphere, the temperature was reduced to a reaction temperature. First, chlorine diluted with nitrogen was allowed to flow through the reaction tube, the chlorine concentration was gradually increased, and finally, a chlorination treatment with the catalyst was performed in a 100% chlorine atmosphere. (Z)-1336mzz (starting material compound) was allowed to flow through the reaction tube at a normal pressure so that the contact time (W/F₀) of (Z)-1336mzz (starting material compound) with the zeolite catalyst was 12 g·sec/cc or 24 g·sec/cc.

The chlorine was used at a Cl_{2/}alkene molar ratio of 2.

The reaction was performed by a gas-phase continuous flow process.

The reactor was heated at 100°C or 200°C to start the chlorination reaction.

After one hour from the start of the chlorination reaction, the distillate that passed through an abatement tower was collected.

After that, mass spectrometry was performed by gas chromatography (trade name: GC-2014, produced by Shimadzu Corporation) in accordance with gas chromatography-mass spectrometry (GC-MS), and structural analysis based on an NMR spectrum was performed using an NMR spectrometer (trade name: 400YH, produced by JEOL).

The results of mass spectrometry and structural analysis confirmed that 336mdd was formed as the target compound.

In Example 1, the conversion rate from (Z)1336mzz (starting material compound) was 45.0 mol%, the selectivity for 336mdd (target compound) was 97.8 mol%, and the selectivity for 316maa was 1.2%.

In Example 2, the conversion rate from (Z) 1336mzz (starting material compound) was 45.1 mol%, the selectivity for 336mdd (target compound) was 98.0 mol%, and the selectivity for 316maa was 1.1%.

In Example 3, the conversion rate from (Z) 1336mzz (starting material compound) was 66.9 mol%, the selectivity for 336mdd (target compound) was 99.6 mol%, and the selectivity for 316maa was 0.1%.

In Example 4, the conversion rate from (Z)1336mzz (starting material compound) was 7.02 mol%, the selectivity for 336mdd (target compound) was 99.6 mol%, and the selectivity for 316 maa was 0.1%.

In Example 5, the conversion rate from (Z) 1336 mzz (starting material compound) was 92.07 mol%, and the selectivity for 336mdd (target compound) was 99.7 mol%, and the selectivity for 316 maa was 0.12%.

The results of the Examples show that when (Z)-1,1,1,4,4,4-hexafluoro-2-butene ((Z)1336mzz) as a starting material compound is subjected to a chlorination reaction in the presence of a zeolite catalyst by adding chlorine to produce 2,3-dichloro-1,1,1,4,4,4-hexafluorobutane (HCFC-336mdd) as the target compound, an alkene can be efficiently subjected to a chlorination reaction and a chlorinated alkane can be produced at a high conversion rate (yield) and with a high selectivity; therefore, the use of the following zeolite catalysts was evaluated as being particularly preferable embodiments.

Among the zeolites, the use of zeolite having a silica/alumina ratio (SiO₂/Al₂O₃ ratio) of 5.5 was evaluated to be particularly preferable.

Among the zeolites, the use of zeolite having an average pore size of 9Å was evaluated to be particularly preferable.

Among the zeolites, the use of zeolite having a cation species (cationic species) of Na⁺ was evaluated to be particularly preferable.

### Comparative Examples: Alumina Catalyst

The chlorination reaction, mass spectrometry, and structural analysis were performed in accordance with the experimental methods in the Examples except that alumina was used as the catalyst.

### Comparative Examples: Silica-Alumina Catalyst

The chlorination reaction, mass spectrometry, and structural analysis were performed in accordance with the experimental methods in the Examples except that silica-alumina was used as the catalyst.

Table 1 below shows the results of the Examples. In Table 1, the contact time (W/F₀) refers to the speed at which the starting material gas is circulated, i.e., the time period during which the catalyst and the starting material gas are in contact with each other.

**Table 1**

| | Kind of catalyst | Pore size (A) | Silica / Alumina ratio | Cationic species | BET specific surface area (m²/g) | Amount of chlorine added, Molar ratio (Cl₂/(Z) 1336mzz) | Contact time W/F₀ (g • sec/cc) | Reaction temperature (°Q | Conversion rate from starting material compound (Z)1336mzz (mol%) | Selectivity of the target compound 336mdd (mol%) | Selectivity of 316maa (mol%) | Other products (mol%) |
|---|---|---|---|---|---|---|---|---|---|---|---|---|
| Ex.1 | Zeolite 1 HSZ330HUD1A | 9 | 6 | Hydrogen | 550 | 2 | 24 | 200 | 45.0 | 97.8 | 1.2 | 1.0 |
| Ex.2 | Zeolite 1 HSZ330HUD1A | 9 | 6 | Hydrogen | 550 | 2 | 12 | 200 | 45.1 | 98.0 | 1.1 | 0.9 |
| Ex.3 | Zeolite 2 NaX | 9 | 2 | Na | - | 2 | 12 | 200 | 66.9 | 99.6 | 0.1 | 0.3 |
| Ex.4 | Zeolite 3 HSZ320NAD1C | 9 | 5.5 | Na | 660 | 2 | 12 | 100 | 7.02 | 99.6 | 0.1 | 0.4 |
| Ex.5 | Zeolite 3 HSZ320NAD1C | 9 | 5.5 | Na | 660 | 2 | 12 | 200 | 92.07 | 99.7 | 0.12 | 0.2 |
| | | | | | | | | | | | | |
| Comp. Ex.1 | Alumina KDH12 | - | - | - | 270 | 2 | 24 | 100 | 0.7 | 98.4 | 0.6 | 1.0 |
| Comp. Ex.2 | Alumina KDH12 | - | - | - | 270 | 2 | 24 | 150 | 1.0 | 88.2 | 10.2 | 1.6 |
| Comp. Ex.3 | Alumina KDH12 | - | - | - | 270 | 2 | 24 | 200 | 2.9 | 91.6 | 6.8 | 1.5 |
| Comp. Ex.4 | Silica Alumina N632L | - | 6.4 | - | 448 | 2 | 12 | 100 | 0.9 | 98.9 | 0.56 | 0.5 |
| Comp. Ex.5 | Silica Alumina N632L | - | 6.4 | - | 448 | 2 | 12 | 200 | 4.9 | 89.9 | 5.2 | 4.9 |

## Claims

1. A method for producing an alkane represented by formula (1) : 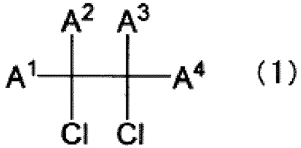
wherein A¹, A², A³, and A⁴ are the same or different and represent a hydrogen atom, a halogen atom, a hydrocarbon group, or a perfluoroalkyl group,
the method comprising the step of subjecting an alkene represented by formula (2): 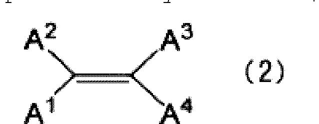
wherein A¹, A², A³, and A⁴ are as defined above,
to a chlorination reaction, the chlorination reaction step being performed using zeolite as a catalyst.

2. The production method according to claim 1, wherein the chlorination reaction is performed in a gas phase.

3. A composition comprising an alkane represented by formula (1): 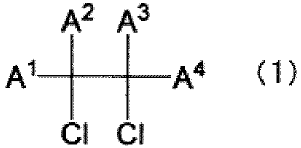
wherein A¹, A², A³, and A⁴ are the same or different and represent a hydrogen atom, a halogen atom, a hydrocarbon group, or a perfluoroalkyl group; and
at least one additional compound comprising a hydrochlorofluorocarbon (HCFC) compound containing at least one chlorine, excluding the alkane represented by formula (1).

4. The composition according to claim 3, wherein the alkane represented by formula (1) is present in an amount of 90 mol% or more and the additional compound is present in an amount of 10 mol% or less, based on the total amount of the composition taken as 100 mol%.

5. The composition according to claim 3 or 4, wherein the additional compound is at least one member selected from the group consisting of monochlorohexafluorobutane, trichlorohexafluorobutane, and tetrachlorohexafluorobutane.

6. The composition according to any one of claims 3 to 5, wherein the composition is for use as an etching gas, a refrigerant, or a heat transfer medium.
